(19) **Europäisches Patentamt**
**European Patent Office**
**Office européen des brevets**

(11) Publication number: **0 525 063 B1**

(12) **EUROPEAN PATENT SPECIFICATION**

(45) Date of publication of patent specification: **20.12.95**   (51) Int. Cl.⁶: **A61K 47/12**

(21) Application number: **91908337.8**

(22) Date of filing: **15.04.91**

(86) International application number:
**PCT/US91/02569**

(87) International publication number:
**WO 91/16077 (31.10.91 91/25)**

(54) **Use of specific fatty acids in the manufacture of a medicament for their Iontophoresis delivery**

(30) Priority: **18.04.90 US 510502**

(43) Date of publication of application:
**03.02.93 Bulletin 93/05**

(45) Publication of the grant of the patent:
**20.12.95 Bulletin 95/51**

(84) Designated Contracting States:
**AT BE CH DE DK ES FR GB GR IT LI LU NL SE**

(56) References cited:
**EP-A- 0 171 742**
**EP-A- 0 351 897**
**WO-A-88/09676**

**DIALOG 1598378, Intern. Pharmaceutical; V. SPRINIVASAN et al., AN 17-02905**

**PHARMACEUTICAL TECHNOLOGY, 1988; R. David, pp. 130-140**

(73) Proprietor: **ALZA CORPORATION**
**950 Page Mill Road**
**P.O. Box 10950**
**Palo Alto**
**California 94303-0802 (US)**

(72) Inventor: **PADMANABHAN, Rama, V.**
**1596 Chatham Avenue**
**Arden Hills, MN 55112 (US)**
Inventor: **SUNRAM, Joan, M.**
**375 - 105th Avenue N.W.**
**Coon Rapids, MN 55433 (US)**

(74) Representative: **Pett, Christopher Phineas et al**
**Frank B.Dehn & Co.**
**Imperial House**
**15-19 Kingsway**
**London WC2B 6UZ (GB)**

Rank Xerox (UK) Business Services
(3.10/3.09/3.3.3)

## Description

Background of the Invention

Iontophoresis is a method for introducing ionic substances into the body. The method utilizes direct electrical current to drive ionized substances, such as drugs, through the intact skin or other body surfaces. This has proven to be very useful in numerous medical applications. U.S. Patent Nos. 3,991,755 issued to Jack A. Vernon et al. and 4,141,359 issued to Stephen C. Jacobsen et al. disclose examples of iontophoretic devices and some applications of the devices. The iontophoretic process has been found to be useful in the administration of lidocaine hydrochloride, hydrocortisone derivatives, acetic acid, fluoride, penicillin, dexamethasone sodium phosphate and many other drugs. Perhaps the best known use of iontophoresis is that of diagnosing cystic fibrosis by using pilocarpine nitrate iontophoresis. The pilocarpine nitrate stimulates sweat production and the sweat is collected and analyzed for its chloride content to detect the presence of the disease.

In iontophoretic devices, two electrodes are used. One electrode, called the active electrode, is the electrode at which the ionic substance is driven into the body. The other electrode, called the indifferent or ground electrode, serves to close the electrical circuit through the body. It will be appreciated by those skilled in the art that the active electrode must hold, contain, or otherwise have available to it a source of the ionic substance. Thus, the active electrode is generally relatively complex compared to the indifferent electrode.

As in the case of methods which attempt to accomplish the passive diffusion of drugs across the skin, the stratum corneum presents the primary barrier to effective drug administration by iontophoresis. This is primarily due to the non-conductive lipids present in this layer, which present a barrier to ionized drugs. This is a particularly serious problem when the transdermal delivery of the ionic form of highly ionized, fat-insoluble (hydrophilic) drugs is attempted.

The use of chemical agents as "penetration enhancers" to improve both passive drug delivery and iontophoresis has been disclosed. Penetration enhancers can disrupt the lipid domain of the stratum corneum, thus lowering the resistance of the skin to ion flow. For example, R. M. Gale et al. (U.S. Patent No. 4,645,502) disclose a device for passive transdermal drug delivery which internally mixes an ionized drug with a penetration enhancer such as an alkyl-substituted sulfoxide, polyoxyethylene glycol esters of fatty acids, and n-methyl pyrrolidone. Certain fatty acids have also been used as penetration enhancers for passive transdermal drug delivery. For example, G. M. Golden et al., in J. Pharm. Sci., 76, 25 (1987) reported that, of a series of octadecenoic acids, only cis-9- and cis-11-octadecenoic acid achieved significant enhancement of salicylic acid through porcine stratum corneum. In addition, B. J. Aungst, Pharm. Res., 6, 244 (1989) summarizes studies indicating that the "kinked structure (the kink due to the cis double bond)" of oleic acid creates gaps in the packed lipid structure of the normal stratum corneum, thus reducing diffusional resistance. For a review of the use of fatty acids as penetration enhancers in passive transdermal therapeutic systems, see K. A. Walters, in Transdermal Drug Delivery, J. Hadgraft et al., eds., Marcel Decker (1989). Theratech, Inc. (published European Patent Application No. 267,617) discloses "penetrating topical compositions" based on the use of a pharmaceutically active agent dissolved in, or admixed with, a binary mixture of oleic acid and ethanol. Up to 50% water can also be included in the compositions, e.g., when they are formulated as gels.

However, a need exists for effective penetration enhancers for the iontophoretic delivery of ionic hydrophilic drugs.

Summary of the Invention

The present invention provides the use of a fatty acid or a physiologically acceptable salt thereof for the manufacture of a composition for use in a method of electrotransported drug delivery through a body surface. The fatty acid for use as a penetration enhancer preferably a $C_{8-18}$ fatty acid, such as oleic acid, lauric acid, capric acid or caprylic acid or a physiologically acceptable salt thereof.

EP-A-435436, which by virtue of ART.54(3)EPC is intermediate prior art citable only in respect of the contracting states AT, BE, CH, DE, DK, FR, GB, IT, LI, LU, NL and SE, describes the use of unsaturated fatty acids as transdermal flux enhancers in iontophoresis.

In a further aspect, the invention thus provides the use of lauric, capric or caprylic acid or physiologically acceptable salts thereof for the manufacture of a composition for use in a method of electrotransported drug delivery through a body surface.

In a preferred embodiment, the penetration enhancer is used in combination with an ionic hydrophilic drug.

The body surface is preferably the intact skin or mucosal membranes of a mammal, but the term "body" also includes plants and the bodies of other animals. Most preferably, both the drug, or drugs, and the fatty acid or fatty acid mixture, are applied as an aqueous solution or dispersion, which can contain an organic co-solvent for the fatty acid. Preferred co-solvents include ethanol, propylene glycol, liquid polyethylene glycols, acetone, tetrahydrofurfuryl alcohol, 1-pyrrolidone, 1-methyl-2-pyrrolidone, N,N-dimethylformamide and the like, with ethanol being most preferred.

The aqueous solution of the fatty acid penetration enchancer and the ionic, hydrophilic drug can be directly contacted with the skin of the mammalian recipient, e.g., a human patient. Alternatively, the aqueous solution of the enhancer and the drug can be pre-gelled, or combined with a polar organic polymer to form an adhesive body. Gels useful for drug matrices for transdermal drug delivery are disclosed in R.M. Gale et al. (U.S. Patent No. 4,645,502). Polar adhesive compositions useful as iontophoretic drug delivery reservoirs as disclosed in Spevak et al. (U.S. Patent No. 4,820,263).

During iontophoresis, application of an electrical current to these "aqueous media" (solutions, gels or adhesives) will cause charged drugs to migrate through the skin of the recipient at a rate that is substantially increased over that observed in the absence of the present fatty acid enhancers. For example, a 0.5% solution of oleic acid in 67:33 ethanol water resulted in a greater than four-fold enhancement of the steady state delivery rate of oxymorphone or hydromorphone hydrochloride through pig skin, in the model system described in detail hereinbelow.

The present use according to the claims also encompasses (a) applying the drug to the skin in combination with the fatty acid enhance, prior to iontophoresis; and (b) applying the fatty acid iontophoretically to the skin, prior to iontophoresis of the drug, through the same area of the skin.

Although the present invention is exemplified in terms of iontophoresis, it is expected that the present fatty acid and fatty acid salt enhancers will also improve the transdermal delivery or transport of drugs which is accomplished by other methods of electrotransport, and such methods are also within the scope of the present invention. The term "electrotransport" refers to methods for transdermal delivery of drugs, whether charged or uncharged, by means of an applied electromotive force to electrolyte-containing reservoir. The particular drug being delivered may be charged or uncharged, depending upon the particular method chosen. When the drug delivered is uncharged, it may be considered delivered by means of electro-osmosis techniques or other electrokinetic phenomenon such as electrohydrokinesis, electro-convection or electrically-induced osmosis. In general, these latter processes for the electrokinetic delivery of uncharged species into a tissue result from the migration of solvent, in which the uncharged species is dissolved, as a result of the application of electromotive force to the electrolyte reservoir. Of course, during the process, some transport of charged species will take place as well.

Brief Description of the Drawings

Figure 1 is a diagrammatic illustration showing an exemplary iontophoretic system.

Figure 2 is a sectional diagrammatic view of a pair of electrodes applied to the body, illustrating the iontophoretic process.

Figure 3 is a sectional view of the iontophoretic diffusion cell according to a preferred embodiment of the invention.

Detailed Description of the Invention

Drugs

The specific drugs used are not critical to the present use according to the claims and as described herein, the term "drug" is to be construed in its broadest sense as a material which is intended to produce some beneficial effect on the organism to which it is applied. Preferably, the-drug used in the present method is hydrophilic and ionizable in an aqueous solvent system. As used herein, a drug in its acid or basic form is considered to be hydrophilic, if the solubility of the drug in mineral oil is less than about 100 $\mu$g/g. Most preferably, the drug is highly ionized in an aqueous solvent. A drug is considered to be "highly ionized" when the percent ionization of the drug in an aqueous drug reservoir is at least about 95%. This occurs when the $pK\alpha$ of the drug differs from the pH of the reservoir by an absolute value of at least 1.3. The $pK\alpha$ of a drug is the pH of an aqueous solution in which 50% of the drug is in the ionized salt form and 50% is in the unionized base or acid form. Since physiological pH of the skin is in the range of

EP 0 525 063 B1

approximately 5.5-7.2, the pKα for the most preferred drugs according to this invention is lower than about 4.2 for acidic drugs and for basic drugs, higher than 8.5 Representative drugs meeting these criteria include, without limitation, acidic drugs such as the sodium or other salts of indomethacin, acetazolamide, methazolamide, and acetylsalisylic acid for example, and salts or acid salts of basic drugs such as naltrexone•HCl, naloxone•HCl, nalbuphine•HCl, oxymorphone•HCl, hydromorphone•HCl, phenylephrine•HCl, chlorphenitramine•maleate, phenylpropanolamine•HCl, clonidine• HCl, dextromethophan•HBr, atropine sulfate, fentanyl citrate, apomorphine sulfate, propranolol•HCl, lidocaine• HCl, tetracycline•HCl, oxytetracycline•HCl, tetracaine•HCl, dibucaine•HCl, terbutaline sulfate, and bromphentramine maleate for example. When the drug is in the form of an acid salt, such as the salt of a basic drug and a pharmaceutically acceptable acid, the drug ions are positively charged, and will generally be delivered from the anode.

The concentration of the drugs in the aqueous media which are employed as the iontophoretic delivery reservoirs, can be varied widely, and will depend to some extent on the rate of delivery through the skin, and on the known effective plasma levels or target tissue levels. Therefore, the appropriate concentration can be preselected by comparing the enhanced rate of delivery attained by the present method, using model systems such as those exemplified hereinbelow, with known in vivo levels which can be attained by iontophoresis without the use of the fatty acid enhancers.

Fatty Acid Penetration Enhancers

The fatty acids useful as penetration enhancers in the present iontophoretic use according to the claims are selected from the group consisting of oleic acid (cis-9-octadecenoic acid), lauric acid (dodecanoic acid), capric acid (decanoic acid) and caprylic acid (octanoic acid). These materials are commercially available, e.g., from Aldrich Chemical Co., Milwaukee, WI. Physiologically acceptable salts of these acids, such as alkali metal salts, can also be employed. However, such salts are not preferred for use in the present method. For example, when 1% of sodium oleate was added to the drug in an ethanol-water mixture, an about two-fold enhancement of drug delivery was obtained. The use of fatty acid salts can permit lower amounts of organic solvents to be used in the aqueous media.

The total concentration of the fatty acid in the aqueous media which are employed as the iontophoretic delivery reservoirs can also be varied widely. For example, it is dependent on its solubility in the organic solvent-water mixture used to prepare the medium, given the need to maintain the salt of the hydrophilic drug in solution, and recognizing that these fatty acids are essentialy water-insoluble. Preferred concentrations are about 0.05-15 wt-%, most preferably about 0.1-10 wt-% of the aqueous medium, e.g., of an aqueous solution comprising about 10-85 vol-% ethanol, ethanol-propylene glycol, or other organic co-solvent.

Iontophoretic Drug Delivery

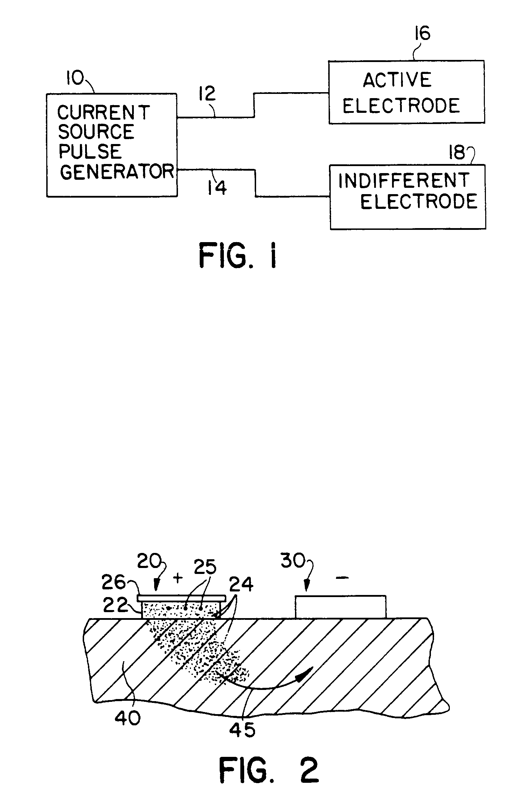

The present use according to the claims can be adapted for use with any of the apparatuses employed for iontophoretic drug delivery. For exemplary purposes herein, an iontophoretic system is illustrated in diagrammatic form in Figure 1. The system includes current source 10 which is electrically coupled through leads 12 and 14 to electrodes 16 and 18, respectively. For purposes of illustration, electrode 16 is labeled the "active" electrode while electrode 18 is labeled the "indifferent electrode," although the positions may be reversed. It is also possible that in some embodiments of electrodes according to the invention that an electrode that is active when the current is flowing in one direction in the system will become an indifferent electrode when the current is flowing the opposite direction in the system. Both electrodes can be "active" electrodes, e.g., when delivery of the drug is by electro-osmosis. Leads 12 and 14 may be wires or they may be any other means for electrically coupling the current source 10 and the electrodes 16 and 18.

Figure 2 shows a sectional view of a pair of electrodes 20 and 30, placed upon body 40 to illustrate the iontophoretic process. Electrode 20 comprises a reservoir element (22) which can hold a solution or a gel in direct or indirect contact with the body, or which can be composed of an adhesive material and a backing sheet (26). A positively charged ionic substance 24(•) is dissolved in the solution, or dispersed throughout the gel or the adhesive layer, preferably in admixture with the penetration enhancer (25)(●). The positively charged ionic substance (24) in reservoir element 22 of electrode 20 is the drug which is to be introduced into the body. Electrodes 20 and 30 may be coupled to a current source, such as 10, through leads, such as 12 and 14 (not shown in Figure 2). The current source 10 may also include a source of electrical pulses, which may be of a type suitable for either nerve or muscle stimulation.

4

The polarity of the current produced by current source 10 may also be reversed by a polarity control (not shown), so that either positively charged ionic substances or negatively charged ionic substances, or both, in alternation, can be delivered from electrode 20. For example, a negatively charged penetration agent could be delivered to the skin, followed by a positively charged ionic drug, and these steps could be repeated, as desired, to maintain the enhanced delivery rate of the drug. See U.S. Patent No. 4,406,658. The leads 12 and 14 may be attached to electrodes 20 and 30 in any conventional manner. When a current is generated by a current source, such as 10, and applied to electrodes 20 and 30 in place upon body 40, a current will flow through body 40 in the direction shown at arrow 45. The current will cause positively charged conic substance 24 to be driven out of electrode 20 into body 40.

A wide variety of active electrodes have been disclosed in the prior art, which can be used for the co-delivery of the present penetration enhancers and the ionically charged drug, so long as they can be admixed in the reservoir element 22.

Inasmuch as the extent of ionized drug migration is a function of the current flow, the rate of iontophoresis increases with a corresponding increase in current. Limitations as to the amount of current are based primarily on considerations of patient safety. Currents of from 0.10 mA to 2-5 mA can be used in the present method. Preferred current densities are about 10-500 $\mu$A/cm$^2$.

Vernon (U.S. Patent No. 3,991,755) discloses several examples of active electrodes. In one example, the electrode comprises a stainless steel wire housed in a plastic sheath which is shaped to safely fit within the ear canal together with a liquid solution which can contain the ionized drug and could contain the present penetration enhancer. The liquid is poured into the ear canal so that it contacts the inner ear and the wire through an opening in the sheath. In another example, the electrode wire housed in a plastic sheath having an opening is placed in a wad of absorbent material which could hold the liquid containing the ionic drug and the penetration enhancer.

U.S. Patent No. 4,141,359 also discloses several embodiments of an active electrode. All of the embodiments include a receptacle for holding either a conducting gel in which the ionic drug and a penetration enhancer can be dissolved, or for holding a sponge which is saturated with the conductive gel in which the ionic drug and the penetration enhancer can be dissolved. The conducting gel drug/enhancer solution communicates with the body tissue through a hole in the receptacle. The receptacle is held in contact with the skin by an adhesive pad surrounding the receptacle or a strap attached to the pad. In other embodiments of the prior art, the hole in the receptacle is covered with a membrane and the ionic subtance is driven through the membrane by the electric current.

An active electrode can be formed by a gauze pad soaked in the solution containing the ionic drug and the enhancer superimposed by several layers of paper towels moistened with tap water and a section of block tin or aluminum foil placed over the moistened towel with the tin or foil connected to the iontophoretic current generator by means of a wire and alligator clip is disclosed in *Acetic Acid Iontophoresis for Calcium Deposits*, by Joseph Kahn in *Physical Therapy*, Vol. 57, No. 6, June, 1977 (pp. 658-659)

Spevak et al. (U.S. Patent No. 4,820,263) disclosed an active electrode which consists essentially of an element composed of an adhesive material mixed with the ionic drug and the penetration enhancer and includes a means for electrically coupling the element to an electrical device. Preferably, the adhesive material is a nonionic, polar material. Webster (U.S. Patent No. 4,383,529) and Ariura et al. (U.S. Patent No. 4,474,570) disclose active electrodes wherein the drug is contained in a hydrophilic polymer/gel.

Although the present penetration enhancers are preferably co-delivered with the ionic drug, the present use according to the claims also encompasses pretreating the skin with the enhancer. Pretreatment of the skin with an effective amount of the enhancer can be accomplished by (a) passive delivery of enhancer by application of the enhancer to the skin in a suitable carrier vehicle, e.g., as a salve or ointment; (b) passive delivery of an effective amount of the enhancer in combination with an adhesive layer which can first make contact with the skin, and which can, at the side distal from the skin, be in contact with the drug reservoir; and (c) iontophoretic delivery of the enhancer from an appropriate vehicle prior to the iontophoretic delivery of the drug to the same area of skin.

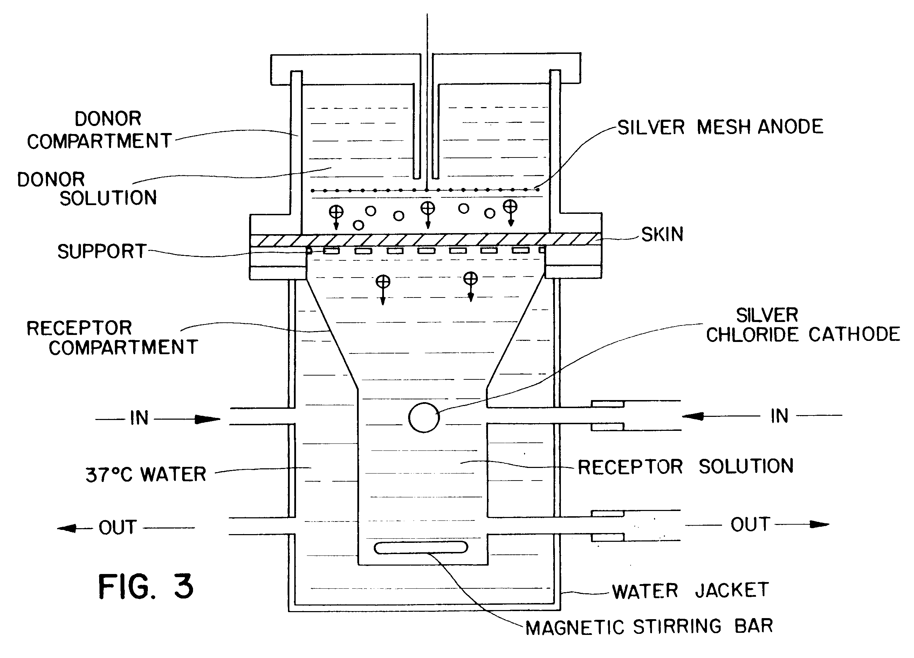

The invention will be further described by reference to the following detailed examples, wherein vertical glass diffusion cells were used. The flow-through diffusion cell (Figure 3) consisted of a donor compartment and a receptor compartment. This cell exposed 5 cm$^2$ of the skin to donor solution, which filled about 75% of the cell volume. A support for the skin exposed 3.26 cm$^2$ of the membrane to the receptor solution and was placed between the two compartments. Either pig skin or human cadaver skin was used. It was placed in the cell dermal side down. The donor compartment was then positioned over the skin and the cell assembly was clamped together. A volume of 7 ml of donor solution was used in each donor compartment.

The receptor compartment contained a small magnetic stirring bar which allowed the contents of the receptor compartment (about 8.5 ml) to be mixed as receptor solution continuously flowed through the cell as indicated. The receptor compartment was surrounded by a water jacket which was maintained at 37°C by circulating warm water therethrough as indicated. The receptor solution completely filled the receptor compartment.

A silver/silver chloride cathode was placed in the receptor compartment. The silver wire of the electrode was threaded out via the inlet port and emerged through silicone tubing. A silver mesh anode was placed in the donor compartment. The silver mesh anode was suspended over the skin by threading the stem of the electrode through the donor cell cover. The anode and cathode wires were connected to one channel of the power source.

A nine-cell flow-through drug delivery system was designed to screen drug candidates for iontophoresis. By selecting various system parameters, each of the nine cells could represent a unique experiment, but generally, each experiment was run in replicates of three or six cells. A multichannel peristaltic pump having multiple cassettes and variable pump speeds was fitted with a specially-made nine-port glass manifold. A nine-cell magnetic stirring console was modified to hold the diffusion cells. Samples were collected using a fraction collector fitted with a nine-port gantry. A heated, circulating water bath maintained the diffusion cells at 37°C. The power source provided nine channels of constant DC current which could be individually adjusted up to 2.0 milliamps (mA) of current.

The flow of receptor solution could be followed through the system. Receptor solution flowed from a reservoir into the pump manifold and through the nine ports, each of which fed into a length of silicone rubber pump tubing (1/16" ID x 1/32" wall). One length of tubing was placed in each pump cassette and receptor solution was pumped at a rate of 3 ml/hr through the inlet port of the receptor compartment of the diffusion cell. There, the receptor solution collected any drug which had been delivered iontophoretically into the receptor compartment. Each of the nine lengths of outlet tubing was held in position over a test tube by a gantry which was mounted to the fraction collector. Receptor solution from each cell was automatically collected in a separate tube for two-hour intervals for the duration of the 66-hour test. The samples were then analyzed for drug content by high-performance liquid chromatography (HPLC).

The iontophoretic drug delivery tests employed two types of skin, dermatomed pig skin and human skin. Pig skin was harvested at a thickness of approximately 0.6 mm using a Padgett dermatome. Human cadaver skin was harvested from the abdominal region at a thickness of approximately 0.3 mm. All skin was stored frozen.

The receptor solution, 0.1 M NaCl (5.84 mg/ml), was prepared in deionized water (DI W). Donor solutions were prepared with model drugs($^\circledR$) at a concentration of 0.1 M in deionized water: oxymorphone hydrochloride was made up to 33.78 mg/ml, and hydromorphone hydrochloride (Dilaudid), was made up to 32.18 mg/ml. The two drugs were used interchangeably since they are similar chemically and previous work has shown equivalent delivery results. The solvent, ethanol (reagent grade), was purchased from Fisher; propylene glycol, 99%, was purchased from Aldrich Chemical Company.

For enhancer studies, the free acid form of the fatty acid (●) was first dissolved in the appropriate amount of ethanol which was then mixed together with the aqueous drug solution such that the final drug content was 0.1 M. Oleic acid (cis-9-octadecenoic acid), 99+% was obtained from Aldrich Chemical Company. Caprylic acid (n-octanoic acid) 99+%; capric acid (n-decanoic acid) 99-100% and lauric acid (dodecanoic acid) 99-100% were obtained from Sigma Chemical Company, St. Louis, MO.

Each experiment consisted of three phases totaling 66 hours. Several schemes are summarized on Table 1 and Table 6.

Table 1

|  |  | Phase | Duration (hrs) | Donor Solution | Current |
|---|---|---|---|---|---|
| Passive Controls | | I | 18 | 0.1 M drug in DI W | Off |
| | | II | 24 | same | Off |
| | | III | 24 | same | Off |
| Controls with Current | | I | 18 | 0.1 M drug in DI W | Off |
| | | II | 24 | same | On |
| | | III | 24 | same | Off |
| Enhancer Studies | | I | 18 | 0.1 M drug in DI W | Off |
| | | II | 24 | 0.1 M drug + enhancer in solvent mixture | On |
| | | III | 24 | same | Off |

6

Phase I was usually an 18-hour passivc (no current) phase. Since drug delivery rates were normally low during this phase, it was used to screen out damaged skin. Generally, the phase I donor solution was an aqueous drug solution, but where the phase I solution included an enhancer, a higher passive drug delivery rate was expected.

Phase II represented the iontophoretic portion of the test and current and resistance measurements across the skin were recorded periodically. Most of the phase II receptor samples were analyzed for drug content so that a delivery profile could be plotted. During iontophoresis, the drug concentration in the receptor compartment increased during the first 6-10 hours and then leveled off; this plateau represented steady state delivery rates. Steady state delivery rates were computed for each cell by knowing the sample concentrations and the volumes collected at each steady state time point. The exact flow rate for each time interval was determined by weighing the sample vials. After measuring the drug concentrations in each vial, the rate of drug delivery was calculated by multiplying the drug concentration by the flow rate (i.e., $\mu$g/ml x ml/hr = $\mu$g/hr). After computing steady state delivery rates with and without enhancers, an enhancement factor was calculated as the ratio of average steady state delivery rate in the presence and absence of enhancer.

During phase III, current was turned off and delivery was allowed to continue for a period of 24 hours. A comparison of delivery rates obtained during phases I and III was used as an indicator of skin damage brought about by the experimental treatment in phase II.

Receptor samples were covered, kept in the dark and refrigerated until they were analyzed for drug by HPLC. The HPLC analysis was performed using a Hewlett Packard 1090A liquid chromatograph equipped with a diode array detector set to 280 nm. A DuPont 5 $\mu$m, $C_{18}$ (150 x 4.6 mm) column was used. The mobile phase was 59% 0.005 M heptanesulfonic acid, 40% methanol and 1% acetic acid; the flow rate was 1 ml/minute. The injection volume was 10 $\mu$l. A standard curve was prepared up to a drug concentration of 1800 mg/ml as the free base equivalent.

Example 1 - Comparison of Fatty Acid Enhancers

Delivery of hydromorphone hydrochloride (HM-HCl) or oxymorphone hydrochloride (Oxy-HCl) across human skin or pig skin, was significantly enhanced by the application of iontophoresis. A typical iontophoretic steady state delivery rate of about 1200 $\mu$g/hr was achieved using a current of 1 mA without the addition of a fatty acid. Further enhancement of this delivery rate was obtained using any of the four fatty acid enhancers, as shown in Table 2.

Table 2.

| Donor Solution Enhancer | Concentration (mg/ml) | (%) | Drug(0.1M) | E/W* | Current (mA) | Steady State Delivery Rate ± sd (μg/hr) | Enhancement Factor |
|---|---|---|---|---|---|---|---|
| None | 0 | (0) | HM-HCl | 0/100 | 0 | 9 ± 2 | 0 |
| None | 0 | (0) | HM-HCl | 0/100 | 1 | 1235 ± 151 | 0 |
| None | 0 | (0) | Oxy-HCl | 67/33 | 1 | 1202 ± 133 | 0 |
| Oleic acid | 5 | (0.5) | Oxy-HCl | 67/33 | 1 | 5597 ± 433 | 4.7 |
| Oleic acid | 10 | (1.0) | HM-HCl | 67/33 | 1 | 5205 ± 498 | 4.3 |
| Caprylic acid | 100 | (10.0) | HM-HCl | 48/52 | 1 | 4289 ± 597 | 3.6 |
| Capric acid | 100 | (10.0) | HM-HCl | 60/40 | 1 | 4674 ± 679 | 3.9 |
| Lauric acid | 100 | (10.0) | HM-HCl | 60/40 | 1 | 4838 ± 766 | 4.0 |

* Ethanol:water (vol:vol).

This data shows about a five-fold enhancement in delivery rate when 0.5% oleic acid and ethanol/water (E/W) was present in the donor solution. The other three enhancers showed about a four-fold enhancement when 10% fatty acid was used in E/W mixtures. Enhancement was not due to the ethanol alone, since the steady state delivery rate of 1202 ± 134 μg/hr for E/W (67/33) compares closely with the rate of 1236 ± 152 μg/hr for the 100% water control. The data also shows no additional enhancement by 1% oleic acid beyond that achieved at the 0.5% level.

Example 2. Effect of Varying Enhancer Concentration

Enhancement with oleic acid was observed at concentrations lower than 0.5% although no enhancement was seen at the lowest concentration tested, 0.025%. These tests were done using pig skin, oxymorphone-HCl and 1 mA of current. Results are shown in Table 3.

Table 3

| Oleic Acid Conc(mg/ml) (%) | E/W | Steady State Delivery Rate ± sd (μg/hr) | Enhancement Factor |
|---|---|---|---|
| 0.25 (0.025) | 48/52 | 916 ± 38 | 0 |
| 0.50 (0.050) | 48/52 | 1583 ± 613 | 1.3 |
| 1.0 (0.10) | 52/48 | 2230 ± 877 | 1.9 |
| 2.0 (0.20) | 56/44 | 3806 ± 512 | 3.2 |
| 2.5 (0.25) | 56/44 | 5020 ± 748 | 4.2 |
| 2.5 (0.25) | 56/44 | 5364 ± 330 | 4.5 |
| 5.0 (0.50) | 67/33 | 5597 ± 433 | 4.7 |

The two identical tests with 0.25% oleic acid (shown in Table 3) were done on different days and show fair agreement, considering the large standard deviations observed in these tests.

Example 3. Effect of Current Variation

In vitro testing showed that enhancement was also observed across a range of lower currents (0.25 to 1.0 mA), using two lower concentrations of oleic acid, 0.2% in 56/44 (E/W) and 0.5% in 62/38 (E/W) with pig skin. The enhanced steady state delivery rates are shown in Table 4. Tests with enhancer may be compared to the control delivery rate with no oleic acid and using human skin. Tests summarized in Table 4 were done using oxymorphone in the donor compartment.

**Table 4.**

Steady State Delivery Rate ± sd (μg/hr) Enhancer (oleic acid)

| Current (mA) | 0 mg/ml (control) | 2 mg/ml (0.2%) | 5 mg/ml (0.5%) |
|---|---|---|---|
| 0 | 27 ± 25 | 1280 ± 221 | 1734 ± 102 |
| 0.25 | 228 ± 55 | 2204 ± 140 | 2832 ± 91 |
| 0.50 | 542 ± 62 | 2597 ± 338 | 3699 ± 222 |
| 0.75 | 910 ± 117 | 3476 ± 465 | 4583 ± 304 |
| 1.0 | 1024 ± 276 | 3806 ± 512 | 5597 ± 433 |

Example 4. Effect of Solvent Variation

A higher concentration of oleic acid (5.0%) was used in two different propylene glycol/ethanol/water (PG/E/W) mixtures. These experiments were carried out in an attempt to lower the percent of ethanol used as a vehicle for fatty acids. High ethanol concentrations in transdermal products can cause unacceptable levels of skin irritation. PG was therefore added as a co-solvent with lower concentrations of ethanol. The control solution contained no oleic acid. The steady state delivery rates are shown in Table 5.

Table 5.

| Oleic Acid (mg/ml) (%) | Solvent | Steady State Delivery Rate ± sd (µg/hr) | Enhancement Factor |
|---|---|---|---|
| 0 (0) | PG/W (65/35) | 835 ± 221 | 0 |
| 50 (5) | PG/E/W (65/20/10) | 3050 ± 371 | 2.5 |
| 50 (5) | PG/E/W (30/45/20) | 5206 ± 427 | 4.3 |

The steady state rate for the controls shows no enhancement from the propylene glycol alone as a solvent, and perhaps a decrease in delivery. Although there was enhancement using PG/E/W mixtures with oleic acid, enhancement was not greater than that which was seen using only 0.5% oleic acid and E/W, 67/33. Also, the solvent mixture with the higher concentration of PG (65%) showed less enhancement than

11

the other solvent mixture having 35% PG.

These tests show that propylene glycol along with lower amounts of ethanol results in a significant enhancement of delivery. However, no additional enhancement was seen using the higher concentration of oleic acid.

Example 5. Pretreatment with Fatty Acid

Several tests were done in which oleic acid was present during phase I, which then was considered a pretreatment phase. All tests employed 10 mg/ml (1%) oleic acid and pig skin. Schemes for these tests are shown in Table 6.

Table 6.

| Scheme | Phase | Donor Solution | Duration (hrs) | Current (mA) |
|---|---|---|---|---|
| Scheme A | I | 0.1 M HM.HCl + enhancer in E/W, 67/33 | 18 | Off |
| | II | same | 24 | On |
| | III | same | 24 | Off |
| Scheme B | I | 0.1 M HM.HCl + enhancer in E/W, 67/33 | 18 | Off |
| | II | 0.1 M HM.HCl in DI W | 24 | On |
| | III | same | 24 | Off |
| Scheme C | I | 0.1 M NaCl + enhancer in E/W, 67/33 | 18 | On |
| | II | 0.1 M HM.HCl in DI W | 24 | On |
| | III | same | 24 | Off |

The test represented by scheme A contained enhancer in all phases. Results from the iontophoretic phase showed that steady state was achieved about four hours earlier than in cells without oleic acid in phase I (see scheme for Enhancer Studies in Table 1). The steady state delivery rates were 5344 ± 541 μg/hr and 5205 ± 499 μg/hr, respectively. Although these rates were essentially the same, the total drug delivery was higher in pretreated cells due to earlier attainment of steady state.

Another test was done in which the enhancer and drug were present in the phase I solution, but no enhancer was present when the phase II current was applied according to scheme B. Results showed that by the end of phase I, delivery reached approximately 1800 $\mu$g/hr due to the presence of 1% oleic acid in the donor solution. The steady state (phase II) delivery rate was 2163 ± 354 $\mu$g/hr. Therefore, delivery was at an intermediate level; greater than that from controls with current and no enhancer (1235 ± 151 $\mu$g/hr) but less than iontophoretic delivery with 1% enhancer (5205 ± 498 $\mu$g/hr).

One additional test was done in which a current was applied to an oleic acid/NaCl mixture in E/W (67/33) during phase I. The purpose of the salt was to provide ions for conductivity. According to scheme C, drug but no enhancer was present in the second phase. The steady state delivery rate was 2164 ± 551 $\mu$g/hr, the same as for the previous test. Total delivery was greater in the previous test due to the already-high delivery at the onset of iontophoresis. Thus, the effect of pretreatment on steady state, but not total delivery, was the same whether pre-treatment included enhancer and drug in the passive mode, (Scheme B), or enhancer but no drug in the phase I iontophoretic mode (Scheme C).

Example 6. Voltage Variations

Typically, the voltage begins to drop when current is applied during phase II, and may continue to fall until about midway through phase II. Some typical changes in voltages are shown in Table 7, along with times at which voltages reached their minimum value. Oxymorphone HCl and pig skin were used in these tests.

Table 7

| Enhancer | Current (mA) | % Drop in Voltage |
|---|---|---|
| 0.2% Oleic acid | 0 | 71 (in 66 hrs) |
| 0.5% Oleic acid | 0 | 74 (in 66 hrs) |
| 0 | 1 | 22 (in 8 hrs w/current) |
| 0.25% Oleic acid | 1 | 42 (in 8 hrs w/current) |
| 0.50% Oleic acid | 1 | 52 (in 8 hrs w/current) |

**Claims**
**Claims for the following Contracting States : AT, BE, CH, DE, DK, FR, GB, IT, LI, LU, NL, SE**

1. The use of lauric, capric or caprylic acid or physiologically acceptable salts thereof for the manufacture of a composition for use in a method of electrotransported drug delivery through a body surface.

2. Use as claimed in claim 1 for the manufacture of an aqueous composition containing an ionic hydrophilic drug and said lauric, capric or caprylic acid or salt thereof and, optionally, an organic co-solvent.

3. Use as claimed in claim 2 for the manufacture of a said composition in solid or gel-like form.

4. Use as claimed in any one of claims 1 to 3 for the manufacture of an aqueous composition comprising a drug, said lauric, capric or caprylic acid or salt thereof and a co-solvent selected from ethanol, propylene glycol, liquid polyethylene glycols, acetone, 1-pyrrolidone, tetrahydrofurfuryl alcohol, 1-methyl-2-pyrrolidone and N,N-dimethylformamide.

5. A pharmaceutical composition for drug delivery by electrotransport through a body surface comprising an aqueous solution or dispersion containing an ionic hydrophilic drug, lauric, capric or caprylic acid or a physiologically acceptable salt thereof and, optionally, an organic co-solvent, said composition further comprising an electrode member disposed in electrical contact with said aqueous solution or dispersion.

6. An electrotransport drug delivery reservoir comprising: a reservoir portion containing an ionic hydrophilic drug in aqueous solution or dispersion together with lauric, capric or caprylic acid or physiologically acceptable salts thereof and optionally together with an organic co-solvent; and a body surface contacting portion which is in electrical contact with said reservoir portion and which comprises said

14

lauric, capric or caprylic acid or salt and, optionally, an adhesive.

7. A reservoir as claimed in claim 6 further comprising electrode means in electrical contact with said reservoir portion.

8. An electrotransport drug delivery kit comprising: a first composition adapted for topical or electrotransport administration to a body surface and comprising lauric, capric or caprylic acid or physiologically acceptable salts thereof; and a second composition adapted for electrotransported administration through a body surface and comprising in aqueous solution or dispersion an ionic hydrophilic drug, optionally together with an acid or salt thereof and optionally together with an organic co-solvent; said first and second compositions being for sequential administration to the same body surface.

**Claims for the following Contracting States : ES, GR**

1. The use of a fatty acid or a physiologically acceptable salt thereof for the manufacture of a composition for use in a method of electrotransported drug delivery through a body surface.

2. Use as claimed in claim 1 of a $C_{8-18}$ fatty acid or a physiologically acceptable salt thereof.

3. Use as claimed in claim 2 of a fatty acid or salt selected from oleic acid, lauric acid, capric acid, caprylic acid and the physiologically acceptable salts thereof.

4. Use as claimed in any one of claims 1 to 3 for the manufacture of an aqueous composition containing an ionic hydrophilic drug and a said fatty acid or salt thereof and, optionally, an organic co-solvent.

5. Use as claimed in claim 4 for the manufacture of a said composition in solid or gel-like form.

6. Use as claimed in any one of claims 1 to 5 for the manufacture of a aqueous composition comprising a drug, a said fatty acid or salt thereof and a co-solvent selected from ethanol, propylene glycol, liquid polyethylene glycols, acetone, 1-pyrrolidone, tetrahydrofurfuryl alcohol, 1-methyl-2-pyrrolidone and N,N-dimethylformamide.

7. A pharmaceutical composition for drug delivery by electrotransport through a body surface comprising an aqueous solution or dispersion containing an ionic hydrophilic drug, a fatty acid or a physiologically acceptable salt thereof and, optionally, an organic co-solvent, said composition further comprising an electrode member disposed in electrical contact with said aqueous solution or dispersion.

8. An electrotransport drug delivery reservoir comprising: a reservoir portion containing an ionic hydrophilic drug in aqueous solution or dispersion together with a fatty acid or a physiologically acceptable salt thereof and optionally together with an organic co-solvent; and a body surface contacting portion which is in electrical contact with said reservoir portion and which comprises a said fatty acid or salt and, optionally, an adhesive.

9. An electrotransport drug reservoir as claimed in claim 8 wherein the fatty acid or salt is selected from oleic acid, lauric acid, capric acid, caprylic acid and the physiologically acceptable salts thereof.

10. A reservoir as claimed in claim 8 or claim 9 further comprising electrode means in electrical contact with said reservoir portion.

11. An electrotransport drug delivery kit comprising: a first composition adapted for topical or electrotransport administration to a body surface and comprising a fatty acid or a physiologically acceptable salt thereof; and a second composition adapted for electrotransported administration through a body surface and comprising in aqueous solution or dispersion an ionic hydrophilic drug, optionally together with an acid or salt thereof and optionally together with an organic co-solvent; said first and second compositions being for sequential administration to the same body surface.

12. An electrotransport drug delivery kit as claimed in claim 11 wherein the fatty acid or salt is selected from oleic acid, lauric acid, capric acid, caprylic acid and the physiologically acceptable salts thereof.

15

EP 0 525 063 B1

**Patentansprüche**

**Patentansprüche für folgende Vertragsstaaten : AT, BE, CH, DE, DK, FR, GB, IT, LI, LU, NL, SE**

1. Verwendung von Laurin-, Caprin- oder Caprylsäure oder ihren physiologisch verträglichen Salzen für die Herstellung einer Zusammensetzung zur Verwendung in einem Verfahren für die Abgabe von Medikamenten durch Transport durch eine Körperoberfläche mit Hilfe von elektrischem Strom.

2. Verwendung gemäß Anspruch 1 für die Herstellung einer wäßrigen Zusammensetzung, die ein dissoziiertes, hydrophiles Medikament und die Laurin-, Caprin- oder Caprylsäure oder ein Salz davon und, gegebenenfalls, ein zusätzliches organisches Lösungsmittel enthält.

3. Verwendung gemäß Anspruch 2 für die Herstellung einer Zusammensetzung in fester oder gelartiger Form.

4. Verwendung gemäß einem der Ansprüche 1 bis 3 für die Herstellung einer wäßrigen Zusammensetzung, die ein Medikament, Laurin-, Caprin- oder Caprylsäure oder ein Salz davon und ein zusätzliches Losungsmittel umfaßt, das aus Ethanol, Proplenglycol, flüssigen Polyethylenglycolen, Aceton, 1-Pyrrolidon, Tetrahydrofurfurylalkohol, 1-Methyl-2-pyrrolidon und N,N-Dimethylformamid ausgewählt ist.

5. Arzneimittel für die Abgabe von Medikamenten durch Transport durch eine Körperoberfläche mit Hilfe von elektrischem Strom, das eine wäßrige Lösung oder Dispersion umfaßt die ein dissoziiertes, hydrophiles Medikament, Laurin-, Caprin- oder Caprylsäure oder ein physiologisch verträgliches Salz davon und, gegebenenfalls, ein zusätzliches organisches Lösungsmittel enthält, wobei die Zusammensetzung außerdem ein Elektrodenelement umfaßt, das so angeordnet ist, daß es mit der wäßrigen Lösung oder Dispersion elektrischen Kontakt hat.

6. Reservoir für die Abgabe von Medikamenten durch Transport mit Hilfe von elektrischem Strom, umfassend: einen Reservoirteil, der ein dissoziiertes, hydrophiles Medikament in wäßriger Lösung oder Dispersion zusammen mit Laurin-, Caprin- oder Caprylsäure oder physiologisch verträglichen Salzen davon und, gegebenenfalls, zusammen mit einem zusätzlichen organischen Lösungsmittel enthält, und einen die Körperoberfläche berührenden Teil, der mit dem Reservoirteil elektrischen Kontakt hat und der Laurin-, Caprin- oder Caprylsäure oder ein Salz und, gegebenenfalls, einen Klebstoff umfaßt.

7. Reservoir gemäß Anspruch 6, das außerdem eine Elektrodenvorrichtung in elektrischem Kontakt mit dem Reservoirteil umfaßt.

8. Kit für die Abgabe von Medikamenten durch Transport mit Hilfe von elektrischem Strom, umfassend: eine erste Zusammensetzung, die für die lokale oder durch elektrischen Strom ermöglichte Anwendung auf einer Körperoberfläche geeignet ist und die Laurin-, Caprin- oder Caprylsäure oder physiologisch verträgliche Salze davon umfaßt, und eine zweite Zusammensetzung, die für die durch elektrischen Strom ermöglichte Verabreichung durch eine Körperoberfläche geeignet ist und die ein dissoziiertes, hydrophiles Medikament in wäßriger Lösung oder Dispersion umfaßt, gegebenenfalls zusammen mit einer Säure oder einem Salz davon und, gegebenenfalls, zusammen mit einem zusätzlichen organischen Lösungsmittel, wobei die erste und die zweite Zusammensetzung zur aufeinanderfolgenden Anwendung auf derselben Körperoberfläche vorgesehen sind.

**Patentansprüche für folgende Vertragsstaaten : ES, GR**

1. Verwendung einer Fettsäure oder eines physiologisch vertraglichen Salzes davon für die Herstellung einer Zusammensetzung zur Verwendung in einem Verfahren für die Abgabe von Medikamenten durch Transport durch eine Körperoberfläche mit Hilfe von elektrischem Strom.

2. Verwendung gemäß Anspruch 1 einer $C_{8-18}$-Fettsäure oder eines physiologisch verträglichen Salzes davon.

3. Verwendung gemäß Anspruch 2 einer Fettsäure oder eines Salzes, ausgewählt aus Ölsäure, Laurinsäure, Caprinsäure, Caprylsäure und ihren physiologisch verträglichen Salzen.

16

**4.** Verwendung gemäß einem der Ansprüche 1 bis 3 für die Herstellung einer wäßrigen Zusammensetzung, die ein dissoziiertes, hydrophiles Medikament und eine Fettsäure oder ein Satz davon und, gegebenenfalls, ein zusätzliches organisches Lösungsmittel enthält.

**5.** Verwendung gemaß Anspruch 4 für die Herstellung einer Zusammensetzung in fester oder gelartiger Form.

**6.** Verwendung gemäß einem der Ansprüche 1 bis 5 für die Herstellung einer wäßrigen Zusammensetzung, die ein Medikament, eine Fettsäure oder ein Satz davon und ein zusätzliches Lösungsmittel umfaßt, das aus Ethanol, Propylenglycol, flüssigen Polyethylenglycolen, Aceton, 1-Pyrrolidon, Tetrahydrofurfurylalkohol 1-Methyl-2-pyrrolidon und N,N-Dimethylformamid ausgewählt ist.

**7.** Arzneimittel für die Abgabe eines Medikaments durch Transport durch eine Körperoberfläche mit Hilfe von elektrischem Strom, das eine wäßrige Lösung oder Dispersion umfaßt, die ein dissoziiertes, hydrophiles Medikament, eine Fettsäure oder ein physiologisch verträgliches Salz davon und, gegebenenfalls, ein zusätzliches organisches Lösungsmittel umfaßt, wobei die Zusammensetzung außerdem ein Elektrodenelement umfaßt, das mit der wäßrigen Lösung oder Dispersion elektrischen Kontakt hat.

**8.** Reservoir für die Abgabe von Medikamenten durch Transport mit Hilfe von elektrischem Strom, umfassend: einen Reservoirteil, der ein dissoziiertes, hydrophiles Medikament in wäßriger Lösung oder Dispersion zusammen mit einer Fettsäure oder einem physiologisch verträglichen Satz davon und, gegebenenfalls, zusammen mit einem zusätzlichen organischen Lösungsmittel enthält, und einen die Körperoberfläche berührenden Teil, der mit dem Reservoirteil elektrischen Kontakt hat und der eine Fettsäure oder ein Satz und, gegebenenfalls, einen Klebstoff umfaßt.

**9.** Reservoir für die Abgabe von Medikamenten durch Transport mit Hilfe von elektrischem Strom gemäß Anspruch 8, in dem die Fettsäure oder das Satz aus Ölsäure, Laurinsäure, Caprinsäure, Caprylsäure und ihren physiologisch verträglichen Salzen ausgewählt ist.

**10.** Reservoir gemäß Anspruch 8 oder Anspruch 9, das außerdem eine Elektrodenvorrichtung in elektrischem Kontakt mit dem Reservoirteil umfaßt.

**11.** Kit für die Abgabe von Medikamenten durch Transport mit Hilfe von elektrischem Strom, umfassend: eine erste Zusammensetzung, die für die lokale oder durch elektrischen Strom ermöglichte Anwendung auf einer Körperoberfläche geeignet ist, und die eine Fettsäure oder ein physiologisch verträgliches Satz davon umfaßt, und eine zweite Zusammensetzung, die für die durch elektrischen Strom ermöglichte Verabreichung durch eine Körperoberfläche geeignet ist, und die ein dissoziiertes, hydrophiles Medikament in wäßriger Lösung oder Dispersion umfaßt, gegebenenfalls zusammen mit einer Säure oder einem Salz davon, und gegebenenfalls zusammen mit einem zusätzlichen organischen Lösungsmittel, wobei die erste und die zweite Zusammensetzung zur aufeinanderfolgenden Anwendung aufderselben Körperoberfläche vorgesehen sind.

**12.** Kit für die Abgabe von Medikamenten durch Transport mit Hilfe von elektrischem Strom gemäß Anspruch 11, bei dem die Fettsäure oder ihr Salz aus Ölsäure, Laurinsäure, Caprinsäure, Caprylsäure und ihren physiologisch verträglichen Salzen ausgewählt ist.

**Revendications**

**Revendications pour les Etats contractants suivants : AT, BE, CH, DE, DK, FR, GB, IT, LI, LU, NL, SE**

**1.** Utilisation d'acide laurique, caprique ou caprylique ou de leurs sels physiologiquement acceptables pour la préparation d'une composition à utiliser dans un procédé de délivrance de médicament électrotransporté au travers d'une surface corporelle.

**2.** Utilisation selon la revendication 1 pour la préparation d'une composition aqueuse contenant un médicament hydrophile ionique et ledit acide laurique, caprique ou caprylique ou leurs sels et éventuellement un co-solvant organique.

3. Utilisation selon la revendication 2 pour la préparation de ladite composition sous une forme solide ou de type gel.

4. Utilisation selon l'une quelconque des revendications 1 à 3 pour la préparation d'une composition aqueuse comprenant un médicament, ledit acide laurique, caprique ou caprylique ou leurs sels et un co-solvant choisi parmi l'éthanol, le propylèneglycol, les polyéthylèneglycols liquides, l'acétone, la 1-pyrrolidone, l'alcool tétrahydrofurfurylique, la 1-méthyl-2-pyrrolidone et le N,N-diméthylformamide.

5. Composition pharmaceutique destinée à la délivrance de médicament par électrotransport au travers d'une surface corporelle, comprenant une solution ou dispersion aqueuse contenant un médicament hydrophile ionique, de l'acide laurique, caprique ou caprylique ou un de leurs sels physiologiquement acceptables et éventuellement un co-solvant organique, ladite composition comprenant de plus un élément électrode disposé en contact électrique avec ladite solution ou dispersion aqueuse.

6. Réservoir de délivrance de médicament par électrotransport comprenant : une partie réservoir contenant un médicament hydrophile ionique en solution ou dispersion aqueuse avec de l'acide laurique, caprique ou caprylique ou leurs sels physiologiquement acceptables et éventuellement avec un co-solvant organique ; ainsi qu'une partie en contact avec la surface corporelle qui est en contact électrique avec ladite partie réservoir et qui comprend ledit acide laurique, caprique ou caprylique ou leur sel et éventuellement un produit adhésif.

7. Réservoir selon la revendication 6 comprenant de plus un moyen de type électrode en contact électrique avec ladite partie réservoir.

8. Kit de délivrance de médicament par électrotransport comprenant : une première composition adaptée pour une administration locale ou par électrotransport à une surface corporelle et comprenant de l'acide laurique, caprique ou caprylique ou leurs sels physiologiquement acceptables; ainsi qu'une deuxième composition adaptée pour une administration par électrotransport au travers d'une surface corporelle et comprenant en solution ou dispersion aqueuse un médicament hydrophile ionique, éventuellement avec un acide ou son sel et éventuellement avec un co-solvant organique ; lesdites première et deuxième compositions étant destinées à une administration successive à la même surface corporelle.

**Revendications pour les Etats contractants suivantes : ES, GR**

1. Utilisation d'un acide gras ou d'un de ses sels physiologiquement acceptables pour la préparation d'une composition à utiliser dans un procédé de délivrance de médicament électrotransporté au travers d'une surface corporelle.

2. Utilisation selon la revendication 1 d'un acide gras en $C_8$-$C_{18}$ ou d'un de ses sels physiologiquement acceptables.

3. Utilisation selon la revendication 2 d'un acide gras ou sel choisis parmi l'acide oléique, l'acide laurique, l'acide caprique, l'acide caprylique et leurs sels physiologiquement acceptables.

4. Utilisation selon l'une quelconque des revendications 1 à 3 pour la préparation d'une composition aqueuse contenant un médicament hydrophile ionique et ledit acide gras ou son sel et éventuellement un co-solvant organique.

5. Utilisation selon la revendication 4 pour la préparation de ladite composition sous une forme solide ou de type gel.

6. Utilisation selon l'une quelconque des revendications 1 à 5 pour la préparation d'une composition aqueuse comprenant un médicament, ledit acide gras ou son sel et un co-solvant choisi parmi l'éthanol, le propylèneglycol, les polyéthylèneglyeols liquides, l'acétone, la 1-pyrrolidone, l'alcool tétrahydrofurfurylique, la 1-méthyl-2-pyrrolidone et le N,N-diméthylformamide.

**7.** Composition pharmaceutique destinée à la délivrance de médicament par électrotransport au travers d'une surface corporelle, comprenant une solution ou dispersion aqueuse contenant un médicament hydrophile ionique, un acide gras ou un de ses sels physiologiquement acceptables et éventuellement un co-solvant organique, ladite composition comprenant de plus un élément électrode placé en contact électrique avec ladite solution ou dispersion aqueuse.

**8.** Réservoir de délivrance de médicament par électrotransport comprenant : une partie réservoir contenant un médicament hydrophile ionique en solution ou dispersion aqueuse avec un acide gras ou un de ses sels physiologiquement acceptables et éventuellement avec un co-solvant organique; ainsi qu'une partie en contact avec la surface corporelle qui est en contact électrique avec ladite partie réservoir et qui comprend ledit acide gras ou sel et éventuellement un produit adhésif.

**9.** Réservoir de délivrance de médicament par électrotransport selon la revendication 8, dans lequel on choisit l'acide ou sel parmi l'acide oléique, l'acide laurique, l'acide caprique, l'acide caprylique et leurs sels physiologiquement acceptables.

**10.** Réservoir selon la revendication 8 ou la revendication 9 comprenant de plus un moyen de type électrode en contact électrique avec ladite partie réservoir.

**11.** Kit de délivrance de médicament par électrotransport comprenant : une première composition adaptée pour une administration locale ou par électrotransport à une surface corporelle et comprenant un acide gras ou un de ses sels physiologiquement acceptables ; ainsi qu'une deuxième composition adaptée pour une administration par électrotransport au travers d'une surface corporelle et comprenant en solution ou dispersion aqueuse un médicament hydrophile ionique, éventuellement avec un acide ou son sel et éventuellement avec un co-solvant organique ; lesdites première et deuxième compositions étant destinées à une administration successive à la même surface corporelle.

**12.** Kit de délivrance de médicament par électrotransport selon la revendication 11, dans lequel on choisit l'acide gras ou sel parmi l'acide oléique, l'acide laurique, l'acide caprique, l'acide caprylique et leurs sels physiologiquement acceptables.

10

12

16

CURRENT
SOURCE
PULSE
GENERATOR

ACTIVE
ELECTRODE

14

18

INDIFFERENT
ELECTRODE

FIG. 1

20 + 25 24

30 −

26
22

40 45

FIG. 2

FIG. 3

Labels: DONOR COMPARTMENT, DONOR SOLUTION, SUPPORT, RECEPTOR COMPARTMENT, 37°C WATER, IN, OUT, SILVER MESH ANODE, SKIN, SILVER CHLORIDE CATHODE, RECEPTOR SOLUTION, IN, OUT, WATER JACKET, MAGNETIC STIRRING BAR

EP 0 525 063 B1